# EUROPEAN PATENT APPLICATION

(11) **EP 3 369 417 A1**
(43) Date of publication of application: **05.09.2018**
(21) Application number: 16859918.1
(22) Date of filing: 27.10.2016
(51) Int. Cl.: A61K 8/60, A61K 31/7105, A61K 48/00, A61P 43/00, A61Q 17/04, C12N 5/22, C12N 15/09

(54) **RNA MOLECULE FOR REPAIRING DNA DAMAGE**

(30) Priority: 30.10.2015 JP 2015215265
(71) Applicant: MIURA, Norimasa, Tottori 6830845 (JP)
(72) Inventor: MIURA, Norimasa, Yonago Tottori 6830845 (JP)
(74) Representative: Gulde & Partner
(86) International application number: PCT/JP2016/081934
(87) International publication number: WO 2017/073689

(57) **Abstract**

The purpose of the present invention is to obtain a novel and effective composition for ameliorating DNA damage. Used is a composition for ameliorating DNA damage, comprising a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1. Also used is a composition for ameliorating DNA damage, comprising a vector encoding a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1.

SEQUENCE LISTING

<110>  NATIONAL UNIVERSITY CORPORATION TOTTORI UNIVERSITY

<120>  RNA molecules to repair DNA damage

<130>  3781TTR

<150>  JP2015-215265
<151>  2015-10-30

<160>  5

<170>  PatentIn version 3.5

<210>  1
<211>  20
<212>  RNA
<213>  Homo sapiens

<400>  1
cuacaaaggg aagcccuuuc                                                   20


<210>  2
<211>  87
<212>  RNA
<213>  Homo sapiens

<400>  2
ucucaagcug ugagucuaca aagggaagcc cuuucuguug ucuaaaagaa aagaaagugc        60

uucucuuugg ugguuacgg uuugaga                                             87


<210>  3
<211>  19
<212>  RNA
<213>  Homo sapiens

<400>  3
aaagugcuuc ucuuuggug                                                     19


<210>  4
<211>  23
<212>  RNA
<213>  Homo sapiens

<400>  4
ucuacaaagg gaagcccuuu cug                                                23


<210>  5
<211>  22
<212>  RNA
<213>  Homo sapiens

<400>  5
aaagugcuuc ucuuuggugg gu                                                 22

## Description

### Technical Field

The present invention relates to compositions for ameliorating DNA damage.

### Background Art

Ultraviolet (UV) light is known to induce DNA damage, and there are several reports on the study in the field of beauty care and cosmetic use.

For instance, the claims of Patent Literature 1 set forth a DNA repair promoter comprising an extract in which the compression strained lees of a fruit of Delaware species (Delaware) are extracted by a polar solvent after cellulase treatment and subsequent polygalacturonase treatment. In addition, the claims of Patent Literature 2 set forth a DNA damage repair promoter for oral application, comprising, as an active ingredient, *Bifidobacterium breve* YIT12272, wherein content of the bacterium is 1×10³ CFU or more as a daily dose. In addition, the claims of Patent Literature 3 set forth a composition for alleviating an ultraviolet irradiation-induced damage, comprising one or more compounds selected from the group of D-proline, etc., and salts thereof

In addition, the claims of Patent Literature 4 set forth an ultraviolet light-protecting agent comprising, as an active ingredient, an extract of leaves of *Chimaphilaumbellata (L.)W. Barton.* In addition, the claims of Patent Literature 5 set forth an anti-dermatopathy agent which suppresses or improves dermatopathy caused by exposure to ultraviolet rays and comprises, as an effective ingredient, a polar solvent extract of *Hippophae rhamnoides L.* of the genus Hippophae belonging to the family Elaeagnaceae, the extract obtained after the *Hippophae rhamnoides L.* is ground and subjected to extraction using a polar solvent, followed by adding celite® and subjecting the resulting mixture to filtration. In addition, the claims of Patent Literature 6 set forth an oral ultraviolet resistance enhancer which comprises, as an active ingredient, gabexate mesilate and can alleviate or inhibit the onset of dermatopathy induced by exposure to ultraviolet radiation.

Meanwhile, the present inventor has reported that miR-520d-5p is effective in inducing stemness characteristics and treating cancer (Patent Literature 7).

### Citation List

### Patent Literature

[Patent Literature 1] Japanese Patent No. 5769448
[Patent Literature 2] Japanese Patent No. 5688376
[Patent Literature 3] Japanese Patent No. 5727364
[Patent Literature 4] Japanese Patent No. 5162145
[Patent Literature 5] Japanese Patent No. 4933768
[Patent Literature 6] Japanese Patent No. 5438369
[Patent Literature 7] Japanese Patent No. 5099571

### SUMMARY OF INVENTION

### Technical Problem

Unfortunately, the technologies of Patent Literatures 1, 2, 4, and 5 are characterized in that prescribed multiple ingredients such as an extract and bacterial cells participate in their effects. Thus, there is room for improvement in view of practical use and availability. Although the technology of Patent Literature 3 involves a specific ingredient, the effect of alleviating ultraviolet irradiation-induced damage is found to be insufficient. Also, the technology of Patent Literature 6 aims at increasing ultraviolet light resistance, so that it is impossible to use the technology for alleviating DNA damage after the UV irradiation. In addition, while the technology of Patent Literature 7 relates to induction of stemness characteristics and treatment of cancer, it is not described that the technology ameliorates DNA damage.

The present invention has been made in view of the above situations. The purpose of the present invention is to provide a novel and effective composition for ameliorating DNA damage.

### Solution to Problem

The present inventor has found that as described in Examples below, when cells which are subject to lethal DNA damage caused by UV irradiation are treated with miR-520d-5p, the DNA damage is ameliorated remarkably. Then, the present inventor has completed the present invention based on the results.

Specifically, an aspect of the present invention provides a composition for ameliorating DNA damage, comprising a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1.

In addition, another aspect of the present invention provides a composition for ameliorating UV light-induced damage, comprising a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1.

In addition, another aspect of the present invention provides a composition for ameliorating DNA damage, comprising a vector encoding a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1.

In addition, another aspect of the present invention provides a composition for ameliorating UV light-induced damage, comprising a vector encoding a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1.

### Advantageous Effects of Invention

According to the present invention, DNA damage or UV light-induced damage can be ameliorated.

### BRIEF DESCRIPTION OF THE DRAWINGS

[FIG. 1] FIG. 1 illustrates how cells after UV irradiation changed
[FIG. 2] FIG. 2 is diagrams showing the flow cytometry results after UV irradiation.
[FIG. 3] FIG. 3 shows the results of measuring collagen production of NHDF cells after UV irradiation.

### DESCRIPTION OF EMBODIMENTS

Hereinafter, embodiments of the present invention will be described in detail. Note that descriptions are not repeated so as to avoid redundancy.

An embodiment of the present invention provides a composition for ameliorating DNA damage, comprising a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1. This composition can ameliorate DNA damage as demonstrated in Examples below. This composition can exert an effect such that cells which are subject to substantial cell death caused by UV irradiation are recovered from DNA damage.

It is preferable that this composition is used after a subject is irradiated with UV light from the viewpoint of increasing the DNA damage-ameliorating effect. It is also preferable that this composition is used in night from the viewpoint of increasing the DNA damage-ameliorating effect. The night includes a period from sunset to bedtime or a period from 18 to 26 o'clock. The period from 18 to 26 o'clock may mean 18, 19, 20, 21, 22, 23, 24, 25, or 26 o'clock. The time may be between any two of the above values.

In an embodiment of the present invention, the "nucleotide sequence set forth in SEQ ID NO: 1" includes a mature miRNA nucleotide sequence
(5'-CUACAAAGGGAAGCCCUUUC-3') of miR-520d-5p.

As used herein, the "composition for amelioration" may be a composition for beauty care use. In addition, examples of the composition for beauty care use include compositions used for cosmetics. In addition, the composition for amelioration may be a composition for research or regenerative medicine use. In addition, the composition for amelioration may be a composition for treatment. In addition, examples of the composition for ameliorating damage include compositions for repairing damage. Examples of the composition for repair include compositions for promoting the repair.

In addition, the composition for ameliorating DNA damage may be a composition for transdermal absorption. The composition for ameliorating DNA damage may be applied onto the skin. Examples of the dosage form of the composition for ameliorating DNA damage include creams and liquids.

As used herein, the "DNA damage" include a cleavage of single-strand DNA, a cleavage of double-strand DNA, and a DNA nucleotide mutation. The DNA damage may be, for example, physical DNA damage. How much the composition ameliorates DNA damage may be evaluated by determining how much the cell growth, which has been decreased by the DNA damage, is increased by treatment using the composition. Alternatively, commercially available kits (e.g., OxiSelect (COSMO BIO co., ltd.), CometAssay Kit (Funakoshi Co., Ltd.)) may be used for the evaluation.

As used herein, the "UV light" includes an electromagnetic wave with a wavelength of from 10 to 400 nm. The wavelength may be, for example, 10, 50, 100, 200, 250, 280, 300, 315, 350, or 400nm. The wavelength may be between any two of the above values. From the viewpoint of promoting beauty care, it is preferable to ameliorating damage caused by near ultraviolet radiation (200 to 380 nm). The near ultraviolet radiation may be divided into UVA (315 to 400 nm), UVB (280 to 315 nm), and UVC (less than 280 nm). The UV irradiation dose may be, for example, 0.01, 0.1, 0.5, 1, 5, 15, 20, 25, or 30 J/cm². The dose may be between any two of the above values.

As used herein, the term "beauty care use" include making one's looks beautiful. Examples of the beauty care use include that the skin damaged by UV irradiation is conditioned.

In an embodiment of the present invention, the "research-use" or "regenerative medicine-use" composition for amelioration may be used to ameliorate damage or growth inhibition of materials (e.g., cells or tissues) used, for example, in research or regenerative medicine, which damage or growth inhibition has been caused by UV irradiation or a DNA damage-inducing chemical. This enables research or regenerative medicine materials to be efficiently used.

As used herein, the term "treatment" includes exerting a prophylactic effect or a symptom-improving effect on a disease of a patient or on one or more symptoms involving the disease. The treatment includes treatment of a disease involving DNA damage, treatment of an aging skin lesion, and treatment of DNA damage caused by an anti-cancer drug. As used herein, the term "anti-cancer drug" includes, for example, a DNA damage-inducing anti-cancer drug.

As used herein, the term "subject" includes a healthy individual, a study subject, or a patient. Examples of the subject include those having UV light damage and those who need amelioration of UV light-induced damage or DNA damage. The subject may be a subject who has received or will receive an anti-cancer drug. Examples of the subject include humans and non-human mammals (e.g., at least one of a mouse, guinea pig, hamster, rat, mouse, rabbit, pig, sheep, goat, cow, horse, cat, dog, marmoset, monkey, and chimpanzee).

As used herein, examples of the "composition" include beauty care preparation, cosmetics, medical cosmetics, therapeutic agents, quasi drugs, and external medicines. This composition may be a composition comprising an active ingredient and one or more cosmetically or pharmaceutically acceptable carriers. The composition may comprise, for example, a whitening component (e.g., arbutin), a moisturizing component (e.g., vaseline, sodium hyaluronate), or a skin-beautifying component (e.g., vitamin C). The composition can be produced by any process known in the art of drug formulation. Examples of the process include: mixing an active ingredient with the above carrier(s). Further, examples of the dosage form of the carrier include, but are not particularly limited to, a solid, a semi-solid, and a liquid. Examples of the dose of the composition include, but are not particularly limited to, 0.01 to 10000 mg/kg body weight per dosing. The dosing interval is not particularly limited, and the composition may be administered, for example, 1 to 3 times during a period of 1 to 30 days. Examples of the dosing route include a transdermal route or an oral route. Examples of the dosage form may include creams, liquids, capsules, tablets, and granules. The dose, the dosing interval, and the dosing method can be appropriately selected depending on the age, body weight, symptom, affected site, etc., of a subject. The composition may be used *in vitro* or *in vivo.* The composition preferably contains a cosmetically effective amount, a therapeutically effective amount, or a dose, which is effective in exerting a desired effect, of an active ingredient. The composition may be included in, for instance, a beauty care or cosmetic container. Examples of the beauty care or cosmetic container include tubes, pump-type containers, wide mouth jars, and narrow mouth bottles.

As used herein, the "polynucleotide" may be, for example, an RNA strand or a DNA strand encoding the RNA strand. This RNA strand may be a translation inhibitory RNA species. Examples of this RNA species include RNA species with a function of inhibiting translation from gene to protein. Examples of this RNA species include miRNA-related molecules and RNAi molecules. The function of inhibiting translation may be checked by quantifying the level of expression of a corresponding RNA strand by real-time RT-PCR. Alternatively, to check the function, the level of expression of the RNA strand may be analyzed by using Northern blotting and/or the level of the resulting protein may be analyzed by using Western blotting as well as the resulting phenotype may be observed. In addition, a plasmid for generating an RNA molecule that can inhibit translation of a specific gene may be purchased from a service company (e.g., TAKARA BIO INC.).

The "polynucleotide" includes a structure in which a plurality of nucleotides or bases, or equivalents are bonded. The concept of the polynucleotide includes, for instance, a chemically-modified polynucleotide, a salt of a polynucleotide, a solvate of a polynucleotide, and a polynucleotide that can bind to a chemical. Examples of the chemical modification include methylation. Examples of the chemical include cellular uptake enhancers (e.g., PEGs or derivatives thereof), labeled tags (e.g., fluorescently labeled tags), and linkers (e.g., nucleotide linkers). As used herein, the concept of the nucleotide includes, for instance, RNA or DNA nucleotides with/without chemical modification. Examples of the equivalents include nucleotide analogs. Examples of the nucleotide analogs include synthetic nucleotides. Examples of the "RNA strand" include a structure in which two or more RNA nucleotides with/without chemical modification or equivalents thereof are bonded. Examples of the polynucleotide include single-strand or double-strand polynucleotides. A nucleotide sequence may be represented by using, for instance, A (adenine), G (guanine), C (cytosine), and T (thymine). Meanwhile, T and U (uracil) are switchable depending on their usage. Nucleotides in such a nucleotide sequence may include A, G, C, and T with/without chemical modification.

The number of nucleotides in the polynucleotide may be, for instance, 15, 16, 17, 18, 19, 20, 21, 22, 23, 24, 25, 30, 40, 50, 60, 70, 80, 90, 100, 200, or 500. The number may be between any two of the above values. The polynucleotide can be synthesized using, for example, a DNA/RNA synthesizer. In addition, the polynucleotide can be purchased from any of service companies (e.g., Invitrogen, Inc., TAKARA BIO INC.) where DNA or RNA polynucleotides can be synthesized. Further, the vector can be synthesized by using service from each company.

The polynucleotide may include the nucleotide sequence set forth in SEQ ID NO: 1. This nucleotide sequence include a mature miRNA of miR-520d-5p. In addition, the polynucleotide may include the nucleotide sequence set forth in SEQ ID NO: 2 (5'-UCUCAAGCUGUGAGUCUACAAAGGGAAGCCCUUUCUGUUGUCUAAAAGAAAA GAAAGUGCUUCUCUUUGGUGGGUUACGGUUUGAGA-3'). This nucleotide sequence includes a full-length sequence of miR-520d-5p. In addition, the polynucleotide may include the nucleotide sequence set forth in SEQ ID NO: 3 (5'-AAAGUGCUUCUCUUUGGUG-3'). This nucleotide sequence may bind complementarily to the nucleotide sequence set forth in SEQ ID NO: 1. In addition, the polynucleotide may include the nucleotide sequence set forth in SEQ ID NO: 4 (5'-UCUACAAAGGGAAGCCCUUUCUG-3'). This nucleotide sequence may function as a guide strand. In addition, the polynucleotide may include the nucleotide sequence set forth in SEQ ID NO: 5 (5'-AAAGUGCUUCUCUUUGGUGGGU-3'). This nucleotide sequence may function as a passenger strand.

As used herein, examples of the "miRNA-related molecules" include miRNA, pri-miRNA, and pre-miRNA. The miRNA-related molecules are known to contribute to inhibiting translation of a target RNA strand. In addition, the translation inhibition is known to be caused by miRNA having an imperfect complementary strand that contains a mismatch(es) and binds partially to the target.

As used herein, the "RNAi molecules" are RNA strands that function as RNAi molecules, and examples include small RNAs that function as siRNA, shRNA, or RNAi molecules. The RNAi molecules can be designed by using, for instance, siDirect 2.0 (Naito et al., BMC Bioinformatics., 2009, Nov. 30; 10:392). In addition, the RNAi molecules may be manufactured by using service from any of service companies (e.g., TAKARA BIO INC.).

As used herein, the "RNAi" involves phenomena such that at least one of siRNA, shRNA, short or long, single or double strand RNA, or a derivative thereof is used to inhibit or silence the function of a target gene or mRNA, etc.

As used herein, the "siRNA" includes a RNA strand(s) that can induce RNAi. Generally speaking, the siRNA double strand may be separated into a guide strand and a passenger strand. Then, the guide strand is incorporated into a RISC. The guide strand incorporated into the RISC is used for recognition of a target RNA. Meanwhile, synthetic RNA is primary used in RNAi research. However, it has been known that endogenous counterparts exist *in vivo.* Each RNA strand may be composed of RNA having 15 or more nucleotides. If the number of nucleotides is 15 or more, the RNA is likely to bind specifically to a target polynucleotide. In addition, each RNA strand may be composed of RNA having 40 or less nucleotides. When the number of nucleotides is 40 or less, there is a lower risk of disadvantageous phenomena such as interferon responses etc.

As used herein, the term "shRNA" includes a single RNA strand that can induce RNAi and can produce a structure (a hairpin-like structure) in which the RNA is bent like a hairpin. Usually, the shRNA is cleaved by a Dicer in a cell to yield siRNA. This siRNA is known to cause a target RNA to be cut. The above shRNA may be composed of 35 or more nucleotides. If the number of nucleotides is 35 or more, a particular hairpin-like structure is likely to be specifically formed in the shRNA. In addition, the above shRNA may be composed of RNA having 100 or less nucleotides. When the number of nucleotides is 100 or less, there is a lower risk of disadvantageous phenomena such as interferon responses etc. In this connection, many pre-miRNAs, the structure and function of which are similar to those of common shRNA, have about 100 or more nucleotides. Accordingly, the length of shRNA may not be 100 bp or less. Nevertheless, the shRNA should be functional.

As used herein, the "small RNA" refers to a relatively small RNA strand(s), and examples include siRNA, shRNA, miRNA-related molecules, antisense RNA, and single- or double-strand, low-molecular-weight RNA.

Translation inhibitory RNA species may contain a 1 to 5-nucleotide overhang at the 5' or 3' end from the viewpoint of increasing the efficiency of inhibiting translation. The number of nucleotides may be 5, 4, 3, 2, or 1. The number may be between any two of the above values. When the translation inhibitory RNA species is double-stranded, a mismatch RNA may be present between the two RNA strands. The number of mismatches may be 1, 2, 3, 4, 5, or 10 or less. The number may be between any two of the above values. The translation inhibitory RNA species may contain a hairpin loop. The number of nucleotides in the hairpin loop may be, for example, 10, 8, 6, 5, 4, or 3. The number may be between any two of the above values. Note that regarding the assignment of each nucleotide sequence, the left side is the 5' end and the right side is the 3' end.

The length of the translation inhibitory RNA species may be, for example, 15, 16, 17, 18, 19, 20, 21, 22, 23, 24, 25, 30, 40, 50, 60, 70, 80, 90, 100, 200, or 500 nucleotides. The length may be between any two of the above values. The length is preferably 15 or more and 100 or less nucleotides from the viewpoint of enhancing the effect of ameliorating DNA damage.

An embodiment of the present invention provides the nucleotide sequences set forth in SEQ ID NO: 1 to 5 such that as long as a desired effect is exerted, (a) a nucleotide sequence containing one or more nucleotide deletions, substitutions, insertions, or additions in any of the above nucleotide sequences may be permitted; and (b) at least one nucleotide sequence selected from the group consisting of nucleotide sequences having 90% or more homology to any of the above nucleotide sequences may be permitted.

As used herein, the above "number of nucleotides" may be, for example, 10, 8, 6, 5, 4, 3, or 2. The number may be any of the values or less. The number is preferably 3 or less from the viewpoint of enhancing the effect of ameliorating DNA damage.

As used herein, the term "90% or more" may mean that the number is, for example, 90, 95, 96, 97, 98, 99, or 100%. The number may be between any two of the above values. The above term "homology" may refer to a ratio of the number of identical nucleotides between two or among a plurality of nucleotide sequences to the total number of nucleotides as calculated in accordance with a method known in the art. Before the calculation of the ratio, nucleotide sequences selected from the group of nucleotide sequences compared are aligned. If the ratio regarding the identical nucleotides needs to be optimized, gaps are inserted in some portions of the nucleotide sequences. An alignment method, a ratio calculation method, a comparison method, and a related computer program have been conventionally well-known in the art (e.g., BLAST, GENETYX). The homology (%) may be represented using values obtained by Blastn with default settings. Alternatively, the homology (%) may be calculated using the formula ((the number of identical nucleotides / the total number of nucleotides in a sequence compared) × 100).

As used herein, the "cell" may be a somatic cell. Examples of this somatic cell include a skin cell and a fibroblast. Examples of a method for introducing a polynucleotide into a cell include viral vector-mediated infection, a calcium phosphate method, lipofection, electroporation, and microinjection. In addition, drug resistance and a cell sorter, for example, may be used to select only the cell containing the polynucleotide.

As used herein, examples of the "vector" include: viral vectors (e.g., lentivirus, adenovirus, retrovirus, or HIV vectors); E. coli-derived plasmids (e.g., pBR322); *Bacillus subtilis-derived* plasmids (e.g., pUB110); yeast-derived plasmids (e.g., pSH19); bacteriophages such as λ phage; and psiCHECK-2, pA1-11, pXT1, pRc/CMV, pRc/RSV, pcDNAI/Neo, pSUPER (OligoEngine, Inc.), BLOCK-it Inducible HI RNAi Entry Vector (Invitrogen, Inc.), and pRNATin-H1.4/Lenti (GenScript, Corp., NJ, USA). The above vectors may each contain, for example, a promoter, a replication origin, and/or an antibiotic resistance gene, which are essential components for expression of the DNA strand. Each vector may be what is called an expression vector.

An embodiment of the present invention provides a composition for ameliorating UV light-induced damage, comprising a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1. This composition can ameliorate UV light-induced cellular damage as demonstrated in Examples below. This composition can exert an effect such that cells which are subject to substantial cell death caused by UV irradiation are recovered from UV light-induced damage.

Another embodiment of the present invention provides a composition for ameliorating DNA damage, comprising a vector encoding a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1. This composition can ameliorate DNA damage as demonstrated in Examples below. This composition can exert an effect such that cells which are subject to substantial cell death caused by UV irradiation are recovered from DNA damage.

Another embodiment of the present invention provides a composition for ameliorating UV light-induced damage, comprising a vector encoding a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1. This composition can ameliorate UV light-induced cellular damage as demonstrated in Examples below. This composition can exert an effect such that cells which are subject to substantial cell death caused by UV irradiation are recovered from UV light-induced damage.

Another embodiment of the present invention provides a beauty care preparation or cosmetic comprising a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1. Another embodiment of the present invention provides a beauty care preparation or cosmetic comprising a vector encoding a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1. The beauty care preparation or cosmetic can ameliorate UV light-induced cellular damage.

Another embodiment of the present invention provides a composition comprising a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1. This composition can ameliorate UV light-induced damage.

Another embodiment of the present invention provides a beauty care method comprising the step of administering, to a subject, a composition comprising a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1. This method can be used to ameliorate the subject's cells with DNA damage. As used herein, the term "amelioration" involves an effect of making a damaged state as close to an original state as possible, and examples include repair and treatment. The step of administering a composition to a subject may include a step of administering the composition to the subject's skin. In addition, the beauty care method may further comprise a step of washing the skin or a step of disinfecting the skin.

Another embodiment of the present invention provides a method for ameliorating DNA damage, comprising the step of administering, to a subject, a composition comprising a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1. Another embodiment of the present invention provides a method for ameliorating UV light-induced damage, comprising the step of administering, to a subject, a composition comprising a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1. Another embodiment of the present invention provides a method for ameliorating DNA damage, comprising the step of administering, to a subject, a composition comprising a vector encoding a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1. Another embodiment of the present invention provides a method for ameliorating UV light-induced damage, comprising the step of administering, to a subject, a composition comprising a vector encoding a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1. Another embodiment of the present invention provides a treatment method comprising the step of administering, to a subject, a composition comprising a vector encoding a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1. Another embodiment of the present invention provides a treatment method comprising the step of administering, to a subject, a composition comprising a vector encoding a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1. The administration step may include a step of applying the composition to the subject's skin. In addition, any of the above methods may further include a step of administering the composition to the skin, a step of washing the skin, or a step of disinfecting the skin. In addition, any of the above methods may further include a step of administering an anti-cancer drug to the subject.

Another embodiment of the present invention provides a method for ameliorating DNA damage or UV light-induced damage, comprising the step of causing a research or regenerative medicine material to contact a composition comprising a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1. Another embodiment of the present invention provides a method for ameliorating DNA damage or UV light-induced damage, comprising the step of causing a research or regenerative medicine material to contact a composition comprising a vector encoding a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1.

Another embodiment of the present invention provides use of a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1 for the manufacture of a composition for ameliorating DNA damage or UV light-induced damage. Another embodiment of the present invention provides use of a vector encoding a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1 for the manufacture of a composition for ameliorating DNA damage or UV light-induced damage.

Another embodiment of the present invention provides a composition for promoting collagen production, comprising a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1. Another embodiment of the present invention provides a composition for promoting collagen production, comprising a vector encoding a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1. These compositions may be used to promote the production of collagen in cells or tissues.

Another embodiment of the present invention provides a composition for suppressing a UV irradiation-mediated decrease in collagen production, comprising a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1. Another embodiment of the present invention provides a composition for suppressing a UV irradiation-mediated decrease in collagen production, comprising a vector encoding a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1. These compositions may be used to suppress a decrease in the production of collagen, which decrease is caused by UV light-induced damage. As used herein, the "composition for promoting collagen production" or the "composition for suppressing a decrease in collagen production" are applicable to cells or tissues. Examples of the cells may include fibroblasts. Examples of the tissues may include skin. The cells or tissues may be cells or tissues that are subject to UV irradiation.

Any documents and (patent or patent application) publications, which are cited herein, are incorporated by reference in its entirety.

As used herein, the term "or" may be used when "at least one" matter listed in the text of specification can be employed. The same applies to the term "or". As used herein, when the wording "between any two of the above values" is indicated, the two values are inclusive in the range. As used herein, the phrase "from A to B" means "A or more and B or less".

As described above, the embodiments of the present invention have been illustrated. These embodiments are examples of the present invention. Accordingly, various configurations other than the above embodiments can be adopted. In addition, combinations among the above-described embodiments can also be employed.

### EXAMPLES

Hereinafter, the present invention is further illustrated by referring to Examples. The present invention, however, is not limited to them.

### <Example 1>

### 1.1. Effect of Recovering from DNA Damage

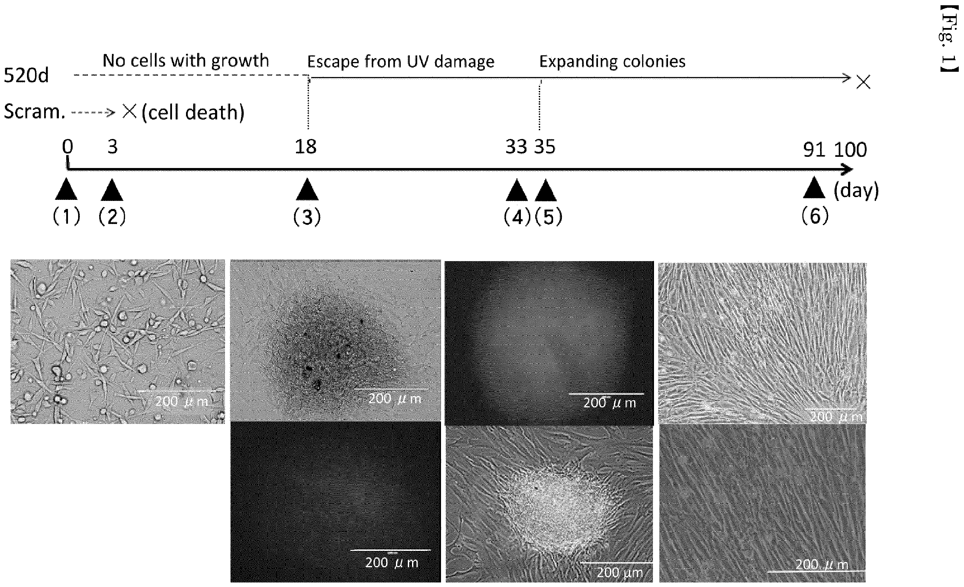

FIG. 1 illustrates how cells after UV irradiation changed. First, NHDF cells (human skin fibroblasts) were cultured on 10-cm culture plates. Next, the cells were irradiated with UV light (302 nm), which strongly causes DNA damage, for 17 min (at 0.5 J/cm²) (FIG. 1(1)). Then, 4-min irradiation was found to be enough for cell death of the NHDF cells.

Before the UV irradiation or 3 days after the UV irradiation, the NHDF cells were infected with a lentivirus (pMIR-520d-5p/GFP) encoding miR-520d-5p (FIG. 1(2)). The nucleotide sequence of a mature miRNA of miR-520d-5p is shown in SEQ ID NO: 1. In addition, 3 days after the UV irradiation, a scrambled control (control nucleic acid) was introduced into the NHDF cells (FIG. 1(2)). The NHDF cells and the NHDF cells having the scrambled control were still subject to cell death after the UV irradiation. In addition, the NHDF cells, to which miR-520d-5p had been introduced before the UV irradiation, were also subject to cell death. Note that the cell death criteria include: (i) during long-term observation, cells do not proliferate for 2 to 3 months; (ii) growth of cells are not detected by MTT assay; (iii) apoptosis kit results show that cells undergo apoptosis; and (iv) flow cytometry results indicate the absence of proliferating cells.

After about 2 weeks, it was observed (FIG. 1(3)) that several spheroid colonies with a low level of GFP expression appeared on a plate having the NHDF cells, to which miR-520d-5p had been introduced after the UV irradiation. The colonies were transferred to a 6-well plate and were cultured. At 1 month or later, each spheroid colony gave rise to fibroblast-like cells (FIG. 1(4)). GFP expression was detected in both the spheroid colonies and the fibroblast-like cells. After trypsin treatment for cell passage, many spheroid cells with a high level of GFP expression appeared (FIG. 1(5)). The cells were subject to cellular senescence at average 75 days after the irradiation. The cells resulted in cell death (FIG. 1(6)). The above results mean that the introduction of miR-520d-5p causes the cells to recover from lethal UV light-induced DNA damage.

The left end photo of FIG. 1 shows a phenotype of NHDF cells before UV irradiation. The second left photos (at week 2 or later) show a phenotype (upper photo) of surviving cells expressing GFP (lower photo). The third left photos (at month 1 or later) show a representative grown spheroid colony with a high level of GFP expression (lower photo). At this time, fibroblast-like cells were produced from the colony (lower photo). The right end photos show fibroblast-like cells grown by about day 80 (upper photo). Small round cells expressing GFP were scattered between the fibroblast-like cells. Note that in this figure, the term "Scram" means that a scrambled control was introduced. The "520d" means that miR-520d-5p was introduced.

### 1.2. Results of Flow Cytometry

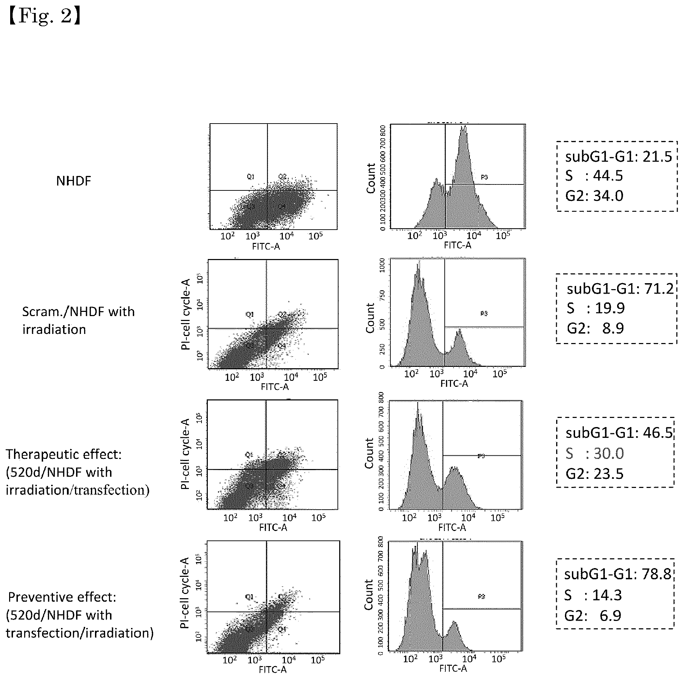

FIG. 2 is diagrams showing the flow cytometry results after UV irradiation. The top panels show the DNA content of NHDF cells. The second top panels show the DNA content of NHDF cells to which a scrambled control was introduced after UV irradiation. The third top panels show the DNA content of NHDF cells to which miR-520d-5p was introduced after UV irradiation. The fourth top panels show the DNA content of NHDF cells to which miR-520d-5p was introduced before UV irradiation. The percentage of each cell cycle phase of the 4 groups was indicated within each dotted rectangular frame on the right side. The NHDF cells, to which the scrambled control had been introduced after UV irradiation, and the NHDF cells, to which miR-520d-5p had been introduced before UV irradiation, were not rescued from lethal damage caused by the UV irradiation. By contrast, the NHDF cells, to which the scrambled control had been introduced after UV irradiation, were found to endure the lethal damage caused by the UV irradiation. In addition, the percentage of the NHDF cells, to which the scrambled control had been introduced after UV irradiation, in S phase (DNA synthesis phase) was 1.5 times higher than that of the NHDF cells, to which the scrambled control had been introduced after the UV irradiation.

### 1.3. Evaluation of Collagen Productivity

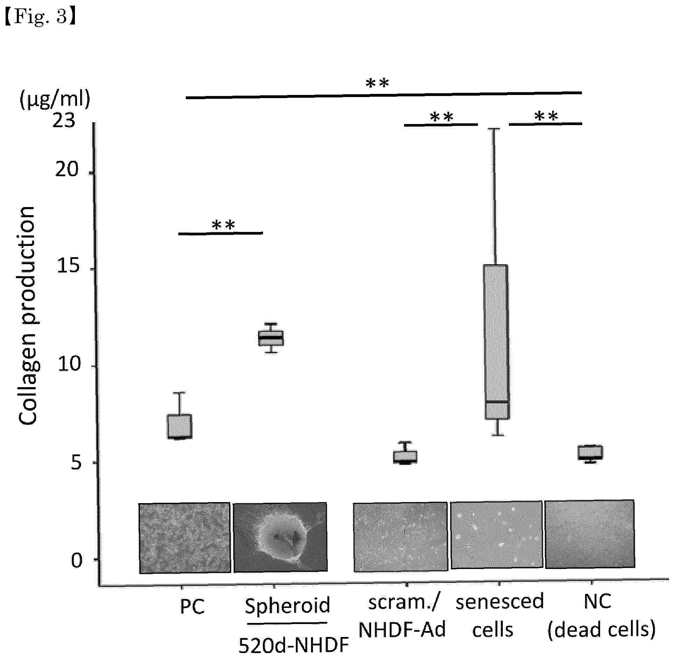

FIG. 3 shows the results of measuring collagen production of NHDF cells after UV irradiation. A spheroid cell population recovered from UV light-induced damage had a markedly high level of collagen productivity. Senesced cells seemed to have a higher level of collagen productivity. In the figure, the "PC" denotes NHDF cells; the "Spheroid" means the NHDF cells (at week 33) to which miR-520d-5p was introduced after UV irradiation; the "**" indicates P < 0.01; and the "NC" denotes dead cells.

The above results have demonstrated that miR-520d-5p elicits the effect of ameliorating DNA damage.

Hereinabove, the present invention has been described based on the Examples. These Examples are absolutely examples. It should be understood by those skilled in the art that various modifications are allowed, and those modifications are also within the scope of the present invention.

## Claims

1. A composition for ameliorating DNA damage, comprising a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1.

2. The composition for ameliorating DNA damage according to claim 1, which is for beauty care or cosmetic use.

3. A composition for ameliorating UV light-induced damage, comprising a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1.

4. A composition for ameliorating DNA damage, comprising a vector encoding a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1.

5. The composition for ameliorating DNA damage according to claim 4, which is for beauty care or cosmetic use.

6. A composition for ameliorating UV light-induced damage, comprising a vector encoding a polynucleotide comprising: a nucleotide sequence set forth in SEQ ID NO: 1; or a nucleotide sequence having 1 to 3 nucleotide deletions, substitutions, insertions, or additions in the nucleotide sequence set forth in SEQ ID NO: 1.
